# NEUE EUROPÄISCHE PATENTSCHRIFT

(11) **EP 0 356 972 B2**
(45) Veröffentlichungstag und Bekanntmachung des Hinweises auf die Entscheidung über den Einspruch: **02.04.1997**
(45) Hinweis auf die Patenterteilung: 08.12.1993
(21) Anmeldenummer: 89115869.3
(22) Anmeldetag: 29.08.1989
(51) Int. Cl.: C07C 227/18

(54) **Verfahren zur Herstellung von beta-Alanindiessigsäure oder ihren Alkalimetall- oder Ammoniumsalzen**
Method for the preparation of beta-alanine-diacetic acid or its alkali metal or ammonium salts
Procédé pour la préparation de bêta alanine diacide acétique ou sels de métaux alcalins ou d'ammonium

(30) Priorität: 02.09.1988 DE 3829859
(43) Veröffentlichungstag der Anmeldung: 07.03.1990
(73) Patentinhaber: BASF Aktiengesellschaft, 67063 Ludwigshafen (DE)
(72) Erfinder: Baur, Richard, Dr., D-6704 Mutterstadt (DE); Gousetis, Charalampos, Dr., D-6700 Ludwigshafen (DE); Trieselt, Wolfgang, Dr., D-6700 Ludwigshafen (DE); Bochnitschek, Werner, Dr., D-6700 Ludwigshafen (DE); Oftring, Alfred, Dr., D-6702 Bad Duerkheim (DE)

(56) Entgegenhaltungen:
- EP-A- 0 265 818
- CA-A- 705 902
- FR-A- 2 355 541
- US-A- 2 195 974
- US-A- 3 260 745
- US-A- 4 133 929
- US-A- 4 563 478
- CHEMICAL ABSTRACTS, vol. 88, no. 23, 5 June 1978, page 542, abstract no. 169605d, Columbus, Ohio, US; & SU - A - 592818
- H.-G. BOIT: "BEILSTEINS HANDBUCH DER ORGANISCHEN CHEMIE", 4th edition, 3rd supplement, vol. 4, part 2, page 1277, 1963, Springer Verlag, Berlin, DE
- HOUBEN-WEYL:" METHODEN DER ORGANISCHEN CHEMIE", 4th edition, vol. XI/1, pages 277-281, 1957, Georg Thieme Verlag, Stuttgart, DE
- V.G. Yashunskii et al., Zh. Vaes Khim Obachestra im D.L. MENDELEEVA 10, (1965), Seite 105
- Allinger et al., Organic Chemistry, Worth, 1971, pp 524-526
- J. Am. Chem. Soc., 1953, vol. 75, p. 2888

## Beschreibung

Die vorliegende Erfindung betrifft ein neues Verfahren zur Herstellung von Alkalimetall- oder Ammoniumsalzen von β-Alanindiessigsäure (Ib).

Die Verbindungen Ib sind ebenso wie die Nitrilotriessigsäure oder die Ethylendiamintetraessigsäure bzw. die Salze dieser Aminopolycarbonsäuren als Komplexbildner von Bedeutung.

Es ist bekannt, β-Alanindiessigsäure durch Umsetzung von Chloressigsäure mit β-Alanin herzustellen (G. Schwarzenbach et al., Helv. Chim. Acta 32 (1949), S. 1184). Weiterhin sind die Cyanomethylierung von β-Alanin zum β-Alanindiacetonitril (V.G. Yashunskii et al., Zh. Vses. Khim. Obschestva im. D.I. Medeleeva 10 (1965), S. 105) und die Umsetzung von Iminodiessigsäure mit Acrylamid zum β-Alanin-N,N-diessigsäure-monoamid (DE-A 27 27 755) bekannt.

Die beiden erstgenannten Verfahren benötigen das relativ schwer zugängliche β-Alanin als Ausgangsstoff und erfordern mehrere Verfahrensstufen, während die Addition der aus Ammoniak, Blausäure und Formaldehyd leicht erhältlichen Iminodiessigsäure an Acrylamid, ebenso wie die Cyanomethylierung des β-Alanins, nur ein Derivat der β-Alanindiessigsäure liefert, aus dem die Säure erst durch anschließende Hydrolyse der Cyanogruppe bzw. der Amidgruppe erhalten wird.

Die SU-A 592 818 offenbart die Herstellung von β-Alanindiessigsäure durch Umsetzung von Iminodiessigsäure und Acrylsäure in wäßriger Natronlauge im stark alkalischen Bereich bei 80 bis 90°C und anschließendes Ansäuern der Reaktionsmischung auf pH 2. Die Ausbeute ist verbesserungsbedürftig.

Der vorliegenden Erfindung lag daher die Aufgabe zugrunde, die Titelverbindungen Ib besser zugänglich zu machen als bisher.

Demgemäß wurde ein Verfahren zur Herstellung von Alkalimetall- oder Ammoniumsalzen von β-Alanindiessigsäure (Ib) durch Umsetzung von Iminodiessigsäure und Acrylsäure miteinander in alkalischem oder stickstoffbasischem wäßrigem Medium gefunden, welches dadurch gekennzeichnet ist, daß man die Umsetzung im pH-Bereich von 7 bis 8 und im Temperaturbereich von 60 bis 100°C während 2 bis 20 Stunden durchführt.

Zweckmäßigerweise werden äquimolare Mengen an Acrylsäure und Iminodiessigsäure eingesetzt. Der Gewichtsanteil der organischen Säuren beträgt, bezogen auf die gesamte wäßrige Mischung, im allgemeinen 20 bis 50, bevorzugt 30 bis 40 Gew.-%. Die Umsetzung setzt bei 20°C spürbar ein und nimmt bei Temperaturen zwischen 60 und 100°C 2 bis 20 Stunden in Anspruch.

Anwendungstechnisch bevorzugt ist der Temperaturbereich von 70 bis 100°C.

Nach beendeter Umsetzung kann die kristalline Säure in Salzform nach den üblichen Verfahren aus der wäßrigen Lösung isoliert werden.

Vorteilhaft wird zunächst unter vermindertem Druck vom Reaktionsgemisch bis zu 50 % Wasser entfernt. Anschließend wird zweckmäßigerweise auf 0 bis 5°C abgekühlt, wobei die β-Alanindiessigsäure in Salzform auskristallisiert, und abfiltriert sowie mit Eiswasser, in Mengen von bis zu 50 % der zur Umsetzung der Iminodiessigsäure mit Acrylsäure als Lösungsmittel eingesetzten Wassermenge, gewaschen. Das Produkt fällt in einer Reinheit von 97 bis 99,5 Gew.-% und in Ausbeuten von 95 bis 98,5 % an.

Aminopolycarbonsäuren sind ganz allgemein wenig wasserlöslich, weshalb in vielen Fällen ihre in Wasser leichter löslichen Alkalimetall- oder Ammoniumsalze anwendungstechnisch bevorzugt sind.

Die Salze der β-Alanindiessigsäure (Ib), in denen alle drei Säurefunktionen neutralisiert sind, sind direkt erhältlich, da man das erfindungsgemäße Verfahren in basischem Medium bei einem pH-Wert von 7 bis 8 durchführt und als neutralisierende Base ein Alkalimetallhydroxid, Ammoniak oder ein organisches Amin zusetzt.

Bevorzugt wird Natronlauge verwendet, jedoch kann man auch andere basische Verbindungen wie Natriumcarbonat, Natriumhydrogencarbonat, Kaliumhydroxid, Kaliumcarbonat oder Ammoniak einsetzen.

Ferner kann man als salzbildende Basen auch primäre, sekundäre oder tertiäre aliphatische organische Amine mit 1 bis 4 C-Atomen in den aliphatischen Resten einsetzen, also beispielsweise Methylamin, Dimethylamin oder Trimethylamin.

Das erfindungsgemäße Verfahren ist sowohl diskontinuierlich als auch kontinuierlich durchführbar und gestattet es, β-Alanindiessigsäure in Form ihrer Alkalimetall- oder Ammoniumsalze auf einfache Weise in hohen Ausbeuten und hoher Reinheit großtechnisch herzustellen.

### Beispiel

Ein Gemisch aus 267 g (2,0 mol) Iminodiessigsäure und 500 ml Wasser wurde mit 50 gew.-%iger NaOH auf pH 7 neutralisiert.

Anschließend wurden innerhalb von 30 min unter Rühren 144 g (2,0 mol) Acrylsäure zugetropft, wobei der pH-Wert der Mischung durch synchrone Zugabe von 50 gew.-%iger NaOH zwischen 7 und 8 gehalten wurde. Anschließend wurde das Reaktionsgemisch 12 h bei 70°C gerührt. Danach wurden unter vermindertem Druck 0,02 bar (0,02 atm) 250 ml Wasser entfernt. Anschließend wurde auf 0°C abgekühlt, wonach das in kristalliner Form ausgefallene Produkt abfiltriert und mit 150 ml Eiswasser gewaschen wurde.
Ausbeute: 525 g (1,94 mol) Trinatriumsalz der β-Alanindiessigsäure = 97 %
Reinheit: 99,2 Gew.-%.

## Patentansprüche

1. Verfahren zur Herstellung von Alkalimetall- oder Ammoniumsalzen von β-Alanindiessigsäure (Ib) durch Umsetzung von Iminodiessigsäure und Acrylsäure miteinander in alkalischem oder Stickstoff basischem wäßrigem Medium, dadurch gekennzeichnet, daß man die Umsetzung im pH-Bereich von 7 bis 8 und im Temperaturbereich von 60 bis 100°C während 2 bis 20 Stunden durchführt.

2. Verfahren nach Anspruch 1, dadurch gekennzeichnet, daß man die Umsetzung im Temperaturbereich von 70 bis 100°C durchführt.

3. Verfahren zur Herstellung des Trinatriumsalzes von β-Alanindiessigsäure in wäßriger Natronlauge nach Anspruch 1 oder 2.

## Claims

1. A process for the preparation of an alkali metal or ammonium salt of β-alaninediacetic acid (Ib) by reacting iminodiacetic acid and acrylic acid with one another in an alkaline aqueous medium or aqueous medium containing a nitrogen base, wherein the reaction is carried out at a pH of from 7 to 8 and at from 60 to 100°C for from 2 to 20 hours.

2. A process as claimed in claim 1, wherein the reaction is carried out at from 70 to 100°C.

3. A process for the preparation of the trisodium salt of β-alaninediacetic acid in aqueous sodium hydroxide solution as claimed in claim 1 or 2.

## Revendications

1. Procédé de préparation de sels de métaux alcalins ou de l'acide d'ammonium β-alaninediacétique (Ib), par la réaction mutuelle de l'acide iminodiacétique et de l'acide acrylique en milieu aqueux alcalin ou basique azoté, caractérisé en ce que l'on entreprend la réaction dans une plage de pH de 7 à 8 et une plage de températures de 60 à 100°C, pendant 2 à 20 heures.

2. Procédé suivant la revendication 1, caractérisé en ce que l'on entreprend la réaction dans une plage de températures de 70 à 100°C.

3. Procédé de préparation du sel trisodique de l'acide β-alaninediacétique dans une solution aqueuse d'hydroxyde de sodium suivant la revendication 1 ou 2.
